(19) Europäisches Patentamt
European Patent Office
Office européen des brevets

(11) Numéro de publication: **0 143 711**
**B1**

(12) # FASCICULE DE BREVET EUROPÉEN

(45) Date de publication du fascicule du brevet: **12.08.87**

(51) Int. Cl.⁴: **C 07 C 97/10**, A 61 K 31/135

(21) Numéro de dépôt: **84402388.7**

(22) Date de dépôt: **23.11.84**

(54) **Dérivés de N-(méthoxyphénacyl)-amine, utilisation notamment en thérapeutique et procédé de préparation.**

(30) Priorité: **25.11.83 FR 8318868**

(43) Date de publication de la demande:
**05.06.85 Bulletin 85/23**

(45) Mention de la délivrance du brevet:
**12.08.87 Bulletin 87/33**

(84) Etats contractants désignés:
**AT BE CH DE FR GB IT LI LU NL SE**

(56) Documents cité:
**EP-A-0 060 954**
**DE-A-1 543 361**
**DE-A-1 593 890**
**FR-A-1 023 815**
**FR-A-2 460 668**
**FR-A-2 503 143**

**Le dossier contient des informations techniques présentées postérieurement au dépôt de la demande et ne figurant pas dans le présent fascicule.**

(73) Titulaire: **LABORATOIRE L. LAFON Société *anonyme dite:*, 1 rue Georges Médéric, F-94701 Maisons- Alfort (FR)**

(72) Inventeur: **Lafon, Louis, 5 rue de l'Alboni, F-75016 Paris (FR)**

(74) Mandataire: **Clisci, Serge, S.A. FEDIT- LORIOT CONSEILS EN PROPRIETE INDUSTRIELLE 38, avenue Hoche, F-75008 Paris (FR)**

EP 0 143 711 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

## Description

La présente invention concerne, en tant que produits industriels nouveaux, des dérivés de N-(méthoxyphénacyl)-amine.

Elle concerne également le procédé de préparation de ces nouveaux produits qui sont utiles en tant que médicaments, d'une part, et en tant qu'intermédiaires de synthèse dans la préparation de dérivés de 2-alkylamino-1-(méthoxyphényl)-1-éthanol également utiles en thérapeutique, d'autre part.

On sait que des dérivés appartenant à la famille des (alkoxyphényl)-aminoalkyl-cétones ont déjà été décrits dans le passé.

On connaît en particulier de US-A-3 895 030 le chlorhydrate de (2,4,6-triméthoxyphényl)-3-pyrrolidino-propyl-cétone qui est un agent vasodilatateur périphérique de référence commercialisé sous le nom de marque de "FONZY-LANE" (Dénomination Commune Internationale: "CHLORHYDRATE de BUFLOMEDIL").

On connaît de l'article de MOED et al., Rec. Trav. Chim., 71, 933-944 (1952) les chlorhydrates de N-(4-méthoxyphénacyl)-tertiobutylamine, N-(4-méthoxyphénacyl)-isopropylamine et N-(3,4-diméthoxyphénacyl)-tertiobutylamine en tant que produits intermédiaires intervenant dans la synthèse de carbinols.

On connaît de US-A-1 680 055 la N-(4-méthoxyphénacyl)-méthylamine en tant que produit utile en thérapeutique et/ou comme intermédiaire de synthèse.

On sait que FR-A-2 503 143 décrit les N-(2,4,6-triméthoxyphénacyl)-isopropylamine et N-(2,4,6-triméthoxyphénacyl)-tertiobutylamine ainsi que leurs sels d'addition en tant que substances agissant sur le système nerveux central eu égard à leurs effets sédatifs.

FR-A-2 460 668 a trait à des dérivés de 2-amino-1-phényl-1-éthanol de formule

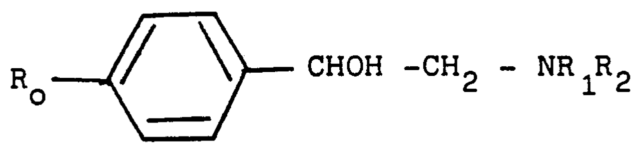

(où notamment $R_0$ est $OCH_3$, $R_1$ est H et $R_2$ est un groupe alkyle en $C_5$- $C_8$ à chaîne hydrocarbonnée ramifiée, i-$C_3H_7$ ou t-$C_4H_9$) et signale, parmi leurs procédés de préparation, celui qui consiste à réduire les composés carbonyle correspondants de formule

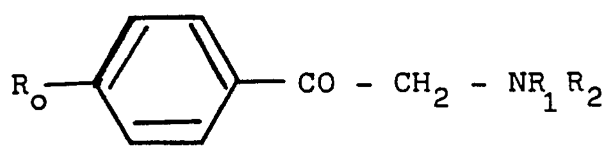

(où $R_0$, $R_1$ et $R_2$ sont définis comme ci-dessus). On sait aussi que des dérivés de N-(fluorophénacyl)-amine de formule

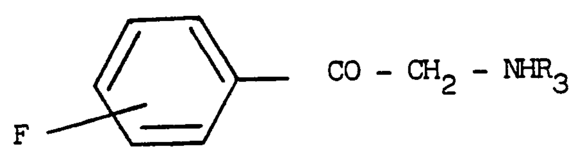

(où $R_3$ est isopropyle ou tertiobutyle) ont été décrits et présentés en tant qu'agents antidépresseurs et/ou sédatifs dans EP-A-0 060 954.

L'ensemble des documents susvisés concerne soit des isomères (cf.MOED et al), soit des analogues (cf US-A-3 895 030, US-A-1 680 055, FR-A-2 460 668, FR-A-2 503 143 et EP-A-0 060 954) des composés suivant l'invention. Chacun de ces documents antérieurs ne décrit (i) les dérivés de N-(méthoxyphénacyl)-amine objets de l'invention tels que visés aux points a), b), c) et d) ci-après, ni (ii) leur utilisation en thérapeutique.

Les dérivés suivant l'invention, qui appartiennent à la famille des N-(méthoxyphénacyl)-alkylamines de formule

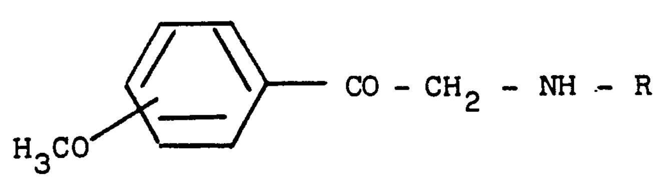 (I)

où R représente le groupe $C(CH_3)_3$ ou $C(CH_3)_2CH_2OH$, sont caractérisés en ce qu'ils sont choisis parmi l'ensemble constitué par

a) la N-(3-méthoxyphénacyl)-tertiobutylamine,
b) la N-(2-méthoxyphénacyl)-tertiobutylamine,
c) la N-(4-méthoxyphénacyl)-N-2-(2-hydroxyméthyl-propyl)-amine, et
d) leurs sels d'addition.

Par sels d'addition, on entend ici, d'une part, les sels d'addition d'acide obtenus par réaction de la base libre de formule I avec les acides minéraux et organiques, et, d'autre part, les sels d'ammonium. Parmi les acides utilisables pour salifier la base de formule I, on peut notamment mentionner les acides chlorhydrique, bromhydrique, acétique, formique, propionique, oxalique, fumarique, maléique, succinique, benzoîque, cinnamique, mandélique, citrique, malique, tartrique, aspartique, glumatique, méthanesulfonique, p-toluènesulfonique. Parmi les composés permettant d'obtenir des sels d'ammonium, on peut notamment citer $ICH_3$ et $ClCH_3$. D'une manière générale les sels d'addition d'acide sont préférés aux sels d'ammonium.

Le produit préféré selon l'invention eu égard à ses propriétés pharmacologiques est le méthanesulfonate de N-(3-méthoxyphénacyl)-tertiobutylamine.

De façon non limitative, un certain nombre de composés selon l'invention a été consigné dans le tableau I ci-après.

**Tableau I**

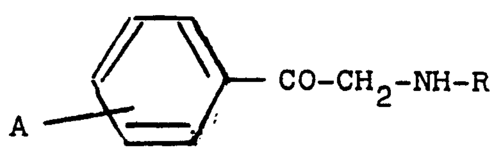

| Produit | N° de code | A | R |
|---|---|---|---|
| Exemple 1 (a) | CRL 40833 | $3-OCH_3$ | $C(CH_3)_3$ |
| Exemple 2 (b) | CRL 40818 | $2-OCH_3$ | $C(CH_3)_3$ |
| Exemple 3 (b) | CRL 40798A | $4-OCH_3$ | $C(CH_3)_2CH_2OH$ |
| Exemple 4 (b) | CRL 40833A | $3-OCH_3$ | $C(CH_3)_3$ |

Notes
(a) : méthanesulfonate
(b) : chlorhydrate

Les composés de formule I peuvent être préparés selon une méthode connue par application de méchanismes réactionnels classiques. Le procédé que l'on préconise consists à faire réagir un bromure de méthoxyphénacyle de formule

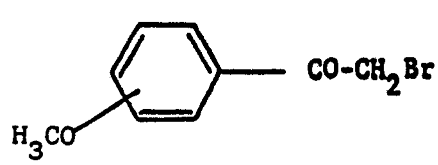

(II)

avec une alkylamine de formulé
$H_2NR$ (III)

(où R est défini comme ci-dessus), en présence d'un solvant inerte notamment choisi parmi l'ensemble constitué par les alcools en $C_1$-$C_2$, le chloroforme, le dioxanne et le tétrahydrofuranne, pendant 0,25 à 2h à une température comprise entre la température ambiante (15-20°C) et la température de reflux du milieu réactionnel à raison d'au moins 3 moles de III pour une mole de II.

Les composés de formule I sont utiles comme intermédiaires de synthèse dans la préparation de dérivés de 2-alkylamino-1-(méchoxyphénacyl)-1-éthanol de formule IV selon le mécanisme

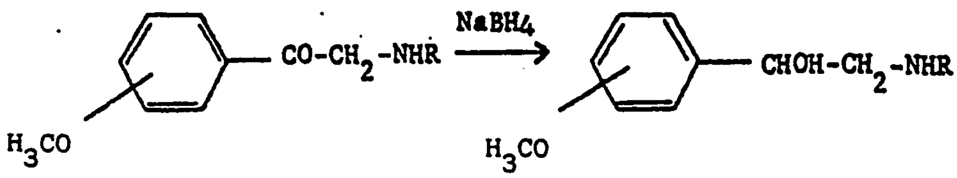


(I) (IV)

(où R est défini comme indiqué ci-dessus) Les composés de formule IV sont utiles en thérapeutique comme indiqué dans la demande française N° 83 18869 déposée par la Demanderesse le 25 novembre 1983.

Les composés selon l'invention présentent des effets bénéfiques sur le système nerveux central et surtout sur le système cardiovasculaire. Ils ont les propriétés communes de diminuer l'agressivité, d'une part, et d'agir en tant que substances vasodilatatrices périphériques, d'autre part.

Selon l'invention on préconise une composition thérapeutique qui est caractérisée en ce qu'elle renferme, en association avec un excipient physiologiquement acceptable, au moins un composé de formule I, selon les points a), b) et c) ci-dessus, ou l'un de ses sels d'addition, en tant que principe actif.

Bien entendu, dans une telle composition le principe actif, qui est choisi parmi l'ensemble constitué par les N-(3-méthoxyphénacyl)-tertiobutylamine, N-(2-méthoxyphénacyl)-tertiobutylamine et N-(4-méthoxyphénacyl)-N-2-(2-hydroxyméthyl-propyl)-amine de formule I et leurs sels d'addition non toxiques, intervient en quantité pharmaceutiquement efficace.

Selon l'invention on préconise l'utilisation d'un dérivé de formule I choisi parmi l'ensemble constitué par la N-(3-métho-xyphénacyl)-tertiobutylamine, la N-(2-méthoxyphénacyl)-tertiobutylamine, la N-(4-méthoxyphénacyl)-N-2-(2-hydroxyméthyl-propyl)-amine et leurs sels d'addition non toxiques, pour l'obtention d'un médicament vasodilatateur périphérique destiné à une utilisation en thérapeutique vis-à-vis des troubles de la circulation.

D'autres avantages et caractéristiques de l'invention seront mieux compris à la lecture qui va suivre d'exemples de préparation et de résultats d'essais pharmacologiques.

**PREPARATION I**

Obtention du méthanesulfonate de N-(3-méthoxyphénacyl)-tertiobutylamine, autre nomenclature: méthanesulfonate de (3-méthoxyphényl)-(tertiobutylaminométhyl)-cétone.

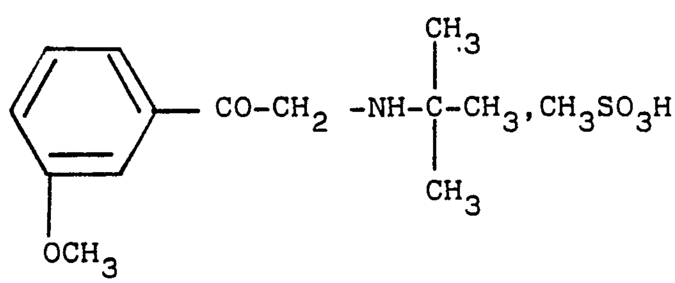

(Exemple 1: N° de code CRL 40 833).

On dissout 50 g (0,218 mole) de bromure de 3-méthoxyphénacyle dans le chloroforme puis coule la solution résultante: dans une solution constituée par 79,6 g (1,09 mole; 114 ml) de tertiobutylamine dans 100 ml de $CH_3OH$. On chauffe au reflux pendant 2 h, évapore à sec, reprend le résidu d'évaporation avec de l'eau, extrait à l'éther, lave la phase éthérée avec de l'eau et la sèche sur $MgSO_4$. On précipite le sel attendu au moyen de 21 g d'acide méthanesulfonique. Par recristallisation dans le mélange acétone-éthanol (1 : 1) v/v, on obtient 10 g (rendement: 16 %) de CRL 40 833. F = 200°C.

**PREPARATION II**

Obtention du chlorhydrate de N-(2-méthoxyphénacyl)-tertiobutylamine.
(Exemple 2; N° de code: CRL 40 818).

On dissout 50 g (0,218 mole) de bromure de 2-méthoxyphénacyle dans 250 ml de chloroforme. On coule cette solution, goutte à goutte dans une solution de: 79,57 g (1,09 mole; 114 ml) de tertiobutylamine dans 100 ml de méthanol. On porte à reflux pendant 2 heures évapore, à sec, reprend à l'eau, extrait à l'éther et précipite le chlorhydrate attendu par l'éthanol chlorhydrique. Par recristallisation dans le mélange acétone-methanol (1 : 1) v/v, on obtient 45 g (rendement: 80 %) de CRL 40 818. F = 264° C (avec décomposition).

Analyse:
N % mesuré. = 5,38 %.
N % théorique = 5,43 %.

**PREPARATION III**

Obtention du chlorhydrate de N-(4-méthoxyphénacyl)-N-2-(2-hydroxyméthyl-propyl)-amine.

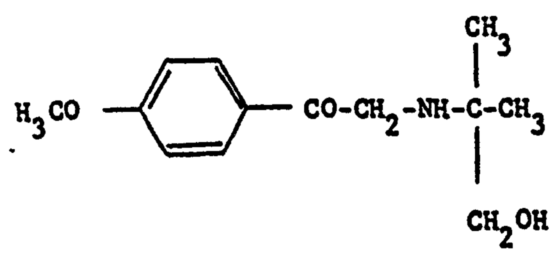

, HCl

(Exemple 3; N° de code: CRL 40 798 A)

On verse 50 g (0,218 mole) de bromure de 4-méthoxyphénacyle (en solution dans 200 ml de chloroforme) dans une solution de 77,6 g (0,872 nole) de 2-(2-hydroxyméthyl-propyl)-amine dans 100 ml de méthanol. On porte au reflux pendant 2 heures, refroidit, évapore à sec, reprend le résidu d'évaporation par 500 ml d'eau, extrait à l'acétate d'éthyle, lave à l'eau et extrait la phase acétate d'éthyle avec un mélange constitué par 500 ml d'eau et 22 ml d'acide HCl concentré (d = 1,19). On lave la phase aqueuse avec $CH_3CO_2C_2H_5$, alcalinise jusqu'à pH 11 avec $K_2CO_3$, extrait avec $CH_3CO_2C_2H_5$, Lave à l'eau, sèche sur $MgSO_4$, filtre. Le filtrat est précipité au moyen d'éthanol chlorhydrique pour donner le CRL 40 798 A attendu

On a résumé les résultats des essais qui ont été entrepris avec les produits selon l'invention.

**A - TOXICITE**

La DL-O (dose maximale non mortelle) chez la souris mâle par voie intrapéritonéale est, pour le CRL 40 833 (exemple 1), supérieure à 256 mg/kg et inférieure à 450 mg/kg.

Les DL-60 par voie intrapéritonéale chez la souris mâle sont de l'ordre de 500 mg/kg pour le CRL 40 833, et de 250 mg/kg pour le CRL 40 818.

**B - ETUDE NEUROPSYCHOPHARMACOLOGIQUE.**

Chez la souris mâle, par voie intrapéritonéale,
on observe que

- en ce qui concerne la motilité, le CRL 40 833 (à partir de la dose de 8 mg/kg) et le CRL 40 818 (à partir de la dose de 64 mg/kg) présentent une motilité accrue et une augmentation de la réactivité, par ailleurs le CRL 40 833 ne provoque pas, à la différence du CRL 40 818, de reprise nette de l'activité motrice chez la souris habituée à son enceinte, n'entraîne pas d'amélioration de la récupération motrice chez la souris dont la motilité a été déprimée à la suite d'un bref séjour dans une enceinte à pression réduite [anoxie hypobare aiguë: dépression de 600 mmHg (soit environ 8 x $10^4$ pascals) en 90 secondes, puis détente en 45 secondes], et n'entraîne pas de modification du délai d'apparition des convulsions et de la mort des souris soumises à une anoxie asphyxique;
- en ce qui concerne l'agressivité intergroupes, le CRL 40 833 (à la dose de 32 mg/kg), et le CRL 40 818 (à partir de la dose de 16 mg/kg) diminuent le nombre de combats, et
- en ce qui concerne les interactions avec la réserpine et l'oxotrémorine, les produits selon l'invention

s'opposent faiblement à l'hypothermie induite par la réserpine et l'oxotrémorine.

## C - ETUDE CARDIOVASCULAIRE.

D'une manière générale, les composés selon l'invention agissent essentiellement en tant que substances vasodilatatrices périphériques, les produits les plus actifs étant les CRL 40 833 et CRL 40 818, le produit le plus intéressant sur le plan thérapeutique étant le CRL 40 833.

### 1°) CHEZ LE CHIEN ANESTHESIE

a) Par voie intraduodénale.

Les essais effectués sur un lot de 4 chiens anesthésiés au nembutal et recevant par voie I.D. des doses de 0,08 à 8 mg/kg de CRL 40 833 montrent que

- le débit artériel fémoral augmente: la dose minimale active est d'environ 0,4 mg/kg, et les doses qui donnent l'effet maximal sont celles qui sont comprises entre 0,8 et 2 mg/kg [en moyenne le débit fémoral en partant de 86 ml/min atteint 156 ml/min (soit + 81 %), la durée de l'effet durant de 2 h à plus de 3 h];
- le débit artériel vertébral augmente: la dose minimale active est d'environ 0,4 mg/kg, et la dose qui donne l'effet maximal est de 0,8 mg/kg [en moyenne le débit vertébral en partant de 51 ml/min atteint 83 ml/min (soit + 57 %), la durée de l'effet étant de 1 à 3 h];
- la fréquence cardiaque augmente à partir de la dose de 0,4 mg/kg, elle atteint ensuite rapidement sa valeur maximale [passage de 197 battements/minute à 217 battements/minute (soit une variation de + 10 %), cette tachycardie modérée se manifeste en général pendant toute la durée de l'essai].

b) Par voie intraveineuse.

Le CRL 40 833 est administré à deux chiens anesthésiés au nembutal aux doses de 0,5 et 1 mg/kg I.V.

A la dose de 0,5 mg/kg sur un chien (pesant 20 kg), le CRL 40 833 fait passer

- le débit fémoral de 87 à 137 ml/min (soit une variation de + 57 %) en 1 h, après 3 h le débit fémoral est encore à 110 ml/min (soit une variation de + 26 % par rapport au même animal avant administration du produit à étudier),
- le débit vertébral de 25 à 55 ml/min (soit une variation de + 120 %) en 0,25 h, après 3 h, le débit vertébral est encore à 40 ml/min (soit une variation de + 60 %);
- la fréquence cardiaque de 180 à 225 battements/minute (soit une variation de + 25 %) en 0,5 h; après 3 h elle est encore de 210 battements/minute (soit une variation de + 16 %), la pression artérielle n'étant pas modifiée.

Une dose supplémentaire de 0,5 mg/kg I.V. fait passer

- le débit fémoral à 122 ml/min (soit une variation de + 40 %), l'augmentation du débit fémoral disparaissant en 1,5 h;
- le débit vertébral à 45 ml/min (soit une variation de + 80 %) pendant environ 1,5 h, et,
- la fréquence cardiaque à 230 battements/minute (soit une variation de + 27 %), la pression artérielle n'étant pas modifiée.

A la dose de 1 mg/kg sur le second chien (pesant 13,5 kg), le CRL 40 833 fait passer

- le débit fémoral de 110 à 150 ml/min (soit une variation de + 36 %) en 25 minutes (l'effet dure plus de 5 h);
- le débit vertébral de 12 à 37 ml/min (soit une variation de + 208 %) en 25 minutes (l'effet se stabilise ensuite à 24 ml/min -soit une variation de + 100 %- pendant 5 h); et,
- la fréquence cardiaque de 150 à 195 battements/minute (soit une variation de + 16 %) après 5 h, la pression artérielle n'étant pas modifiée.

c) Par administration intra-artérielle fémorale.

Le CRL 40 833 est administré par perfusion dans l'artère fémorale d'une patte en 0,5 h, à un chien (11 kg) à 0,1 mg/kg puis à 0,5 mg/kg, et à deux chiens (12 kg et respectivement 13 kg) à la dose de 1 mg/kg puis 1 mg/kg. On observe que le produit entraîne une vasodilatation fémorale sur la patte non perfusée à partir de la dose de 0,6 mg/kg, qu il entraîne par ailleurs une vasodilatation vertébrale, une tachycardie et, sur les deux pattes, un effet anti-isoprénaline.

En particulier le débit fémoral de la patte non perfusée passe de 40 ml/min à 80 ml/min après administration de 0,1 mg/kg + 0,5 mg/kg, et en moyenne de 120 ml/min à 152 ml/min après administration de 1 mg/kg + 1 mg/kg. En même temps le débit vertébral passe de 25 ml/min à 90 ml/min à la dose de 0,1 mg/kg + 0,5 mg/kg, et la fréquence cardiaque passe de 180 à 220 battements/minute à la dose de 0,1 mg/kg + 0,5 mg/kg et en moyenne de 162 à 195 battements/minute à la dose de 1 mg/kg + 1 mg/kg

### 2°) CHEZ LE CHIEN EVEILLE

a) Par voie intraveineuse

Deux chiens de race Labrador sont habitués à rester calmes sur une table; ils reçoivent en dose cumulée: 0,01 mg/kg, 0,03 mg/kg, 0,1 mg/kg, 0,3 mg/kg et 1 mg/kg I.V. de CRL 40 833. On détermine la dose qui augmente la fréquence de 50 battements/minute: DE-50 battements = 0,33 mg/kg et celle qui augmente la

fréquence de 100 battements/minute:
DE-100 battements = 1 g/kg.
b) Par voie buccale
Deux autres chiens de race Labrador reçoivent par voie buccale une dose unique de 1 mg/kg de CRL 40 833. Le CRL 40 833, à la dose de 1 mg/kg V.B., diminue la pression artérielle de 28 % (128-100 mmHg) et augmente la fréquence cardiaque de 98 % (94-187 battements/minute) en 1 heure. Ces effets diminuent ensuite. Après 24 heures la fréquence cardiaque a repris sa valeur témoin alors que la pression artérelle est encore diminuée de 14 %.

3°) **TRACHEE ISOLEE DE COBAYE**

Le CRL 40 833 est étudié par comparaison avec l'isoprénaline sur 6 trachées isolées de cobaye contractées au Carbachol ($3x10^{-7}$ M). On mesure l'activité intrinsèque, α, du CRL 40 833 c'est-à-dire son action maximale par rapport à l'action maximale de l'isoprénaline prise comme unité. On calcule le $pD_2$ qui mesure l'affinité de l'agoniste pour le récepteur et la $CE_{50}$ (concentration qui donne 50 % de l'effet maximal obtenu avec la substance considérée). Les résultats de ces essais sont consignés dans le tableau II qui suit:

**Tableau II**

TRACHEE ISOLEE DE COBAYE

| Paramètres | Isoprénaline | CRL 40 833 |
|---|---|---|
| α | 1 | 0,84 |
| $pD_2$ | 6,85 ± 0,064 | 4,11 ± 0,107 |
| $CE_{50}$ | $1,48 \times 10^{-7}$ M | $8,5 \times 10^{-5}$ M (a) |
| Note<br>(a) soit un rapport $CE_{50}$ CRL 40 833 / $CE_{50}$ isoprénaline de 574. | | |

En conclusion sur la trachée isolée de cobaye, le CRL 40 833 s'est avéré être 574 fois moins $\beta^{+}{}_2$ que l'isoprénaline.

4°) **RAT ISOLE GENETIQUEMENT HYPERTENDU**

16 rats génétiquement hypertendus sont répartis au hasard en 2 groupes de 8 animaux, un groupe reçoit du sérum physiologique par voie buccale (0,2 ml/kg/j), l'autre, le CRL 40 833, à la dose de 10 mg/kg/j, par voie buccale. Le traitement dure 9 jours. Le 9e jour, sous anesthésie au chloral, leur artère fémorale est cathétérisée et le 10e jour, soit 24 heures après la dernière prise, on mesure leur pression artérielle et leur fréquence cardiaque; puis, les aniamux reçoivent à 2 heures d'intervalle par voie buccale, soit du sérum physiologique, soit 10 mg/kg de CRL 40 833, selon le groupe auquel ils appartiennent.
Les moyennes des valeurs des pressions artérielles et des fréquences cardiaques des deux groupes ne diffèrent pas. L'administration de 10 mg/kg de CRL 40 833 à 2 heures d'intervalle entraîne une tachycardie lors des deux administrations (de 370 à 420 et 410 battements/minute, statistiquement significatif à 1 %) d'une durée inférieure à heure, sans effet significatif sur la pression artérielle.
En clinique on a obtenu de bons résultats en administrant le CRL 40 833 en tant qu'agent vasodilatateur, notamment chez l'homme adulte, par voie orale, à raison de 3 à 4 gélules (renfermant chacune 50 mg de CRL 40 833) par jour.

**Revendications**

pour les états contractants: BE, CH, DE, FR, GB, IT, LI, LU, NL, SE.

1. Dérivé appartenant à la famille des N-(méthoxyphénacyl)-alkylamines de formule

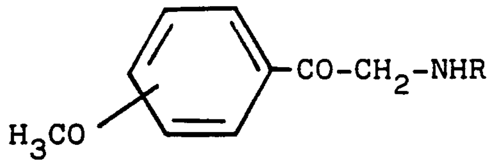

(I)

où R représente un groupe $C(CH_3)_3$ ou $C(CH_3)_2\ CH_2OH$, caractérisé en ce qu'il est choisi parmi l'ensemble constitué par

a) la N-(3-méthoxyphénacyl)-tertiobutylamine,
b) la N-(2-méthoxyphénacyl)-tertiobutylamine,
c) la N-(4-méthoxyphénacyl)-N-2-(2-hydroxyméthyl-propyl)-amine,
d) et leurs sels d'addition.

2. Méthanesulfonate de N-(3-méthoxyphénacyl)-tertiobutylamine.

3. N-(2-méthoxyphénacyl)-tertiobutylamine et ses sels d'addition.

4. N-(4-méthoxyphénacyl)-N-2-(2-hydroxyméthyl-propyl)-amine et ses sels d'addition.

5. Composition thérapeutique caractérisée en ce qu'elle renferme, en association avec un excipient physiologiquement acceptable, au moins un dérivé de N-(méthoxyphénacyl)-alkylamine de formule I selon l'une quelconque des revendications 1 à 4 et choisi parmi l'ensemble constitué par les N-(3-méthoxyphénacyl)-tertiobutylamine, N-(2-méthoxyphénacyl)-tertiobutylamine, N-(4-méthoxyphénacyl)-N-2-(2-hydroxyméthyl-propyl)-amine et leurs sels d'addition non toxiques.

6. Utilisation d'un dérivé de N-(méthoxyphénacyl) -alkylamine choisi parmi l'ensemble constitué par

a) la N-(3-méthoxyphénacyl)-tertiobutylamine,
b) la N-(2-méthoxyphénacyl)-tertiobutylamine,
c) la N-(4-méthoxyphénacyl)-N-2-(2-hydroxyméthyl-propyl) amine, et
d) leurs sels d'addition non toxiques, pour l'obtention d'un médicament vasodilatateur périphérique destiné à une utilisation thérapeutique vis-à-vis des troubles de la circulation.

7. Utilisation selon la revendication 6, caractérisée en ce que le dérivé de N-(méthoxyphénacyl)-alkylamine est le méthanesulfonate de N-(3-méthoxyphénacyl)-tertiobutylamine.

8. Procédé de préparation d'un dérivé de N-(méthoxyphénacyl)-alkylamine de formule I selon la revendication 1 et choisi parmi l'ensemble constitué par

a) la N-(3-méthoxyphénacyl)-tertiobutylamine,
b) la N-(2-méthoxyphénacyl)-tertiobutylamine,
c) la N-(4-méthoxyphénacyl)-N-2-(2-hydroxyméthyl-propyl)-amine, et
d) leurs sels d'addition,

ledit procédé étant caractérisé en ce que l'on fait réagir un bromure de méthoxyphénacyle de formule

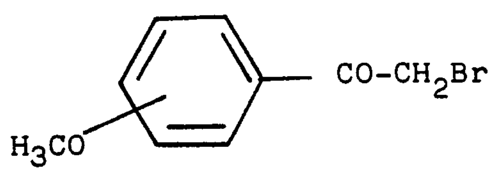

(II)

avec une alkylamine de formule

$H_2NR$ (III)

(où R est défini comme ci-dessus), en présence d'un solvant inerte notamment choisi parmi l'ensemble constitué par les alcools en $C_1$-$C_2$, le chloroforme, le dioxanne et le tétrahydrofuranne, pendant 0,25 à 2 h, à une température comprise entre la température ambiante (15-20°C) et la température de reflux du milieu réactionnel à raison d'au moins 3 moles de III pour une mole de II.

**Revendications**

pour l'état contractant: AT.

1. Procédé de préparation d'un dérivé de N-(méthoxyphénacyl)-alkylamine de formule

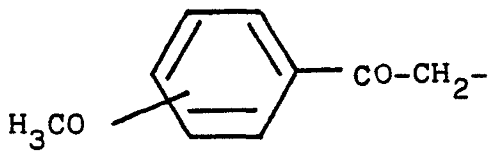
(I)

où R représente un groupe $C(CH_3)_3$ ou $C(CH_3)_2$ $CH_2OH$, et choisi parmi l'ensemble constitué par
a) la N-(3-méthoxyphénacyl)-tertiobutylamine,
b) la N-(2-méthoxyphénacyl)-tertiobutylamine,
c) la N-(4-méthoxyphénacyl)-N-2-(2-hydroxyméthyl-propyl)-amine,
d) et leurs sels d'addition,
ledit procédé étant caractérisé en ce que l'on fait réagir un bromure de méthoxyphénacyle de formule

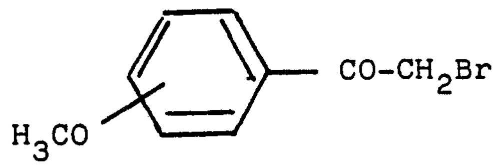
(II)

avec une alkylamine de formule
$H_2NR$ (III)
(où R est défini comme ci-dessus), en présence d'un solvant inerte notamment choisi parmi l'ensemble constitué par les alcools en $C_1$-$C_2$, le chloroforme, le dioxanne et le tétrahydrofuranne, pendant 0,25 à 2 h, à une température comprise entre la température ambiante (15-20°C) et la température de reflux du milieu réactionnel à raison d'au moins 3 moles de III pour une mole de II.

2. Procédé selon la revendication 1 pour préparer la N-(3-méthoxyphénacyl)-tertiobutylamine, caractérisé en ce que l'on fait réagir le bromure de 3-méthoxyphénacyle avec la tertiobutylamine.

**Patentansprüche**

für die Vertragsstaaten: BE, CH, DE, FR, GB, IT, LI, LU, NL, SE.

1. Zur Klasse der N-(Methoxyphenacyl)-alkylamine der Formel

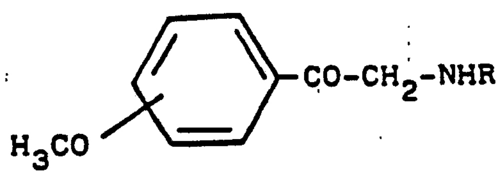
(I),

in welcher R eine $C(CH_3)_3$- oder $C(CH_3)_2CH_2OH$-Gruppe bedeutet, gehörendes Derivat, dadurch gekennzeichnet, daß es aus der aus
a) N-(3-Methoxyphenacyl)-tertiobutylamin,
b) N-(2-Methoxyphenacyl)-tertiobutylamin,
c) N-(4-Methoxyphenacyl)-N-2-(2-hydroxymethyl-propyl)-amin und
d) ihren Additionssalzen gebildeten Gruppe ausgewählt ist.

2. Methansulfonat von N-(3-Methoxyphenacyl)-tertiobutylamin.

3. N-(2-Methoxyphenacyl)-tertiobutylamin und seine Additionssalze.

4. N-(4-Methoxyphenacyl)-N-2-(2-hydroxymethyl-propyl)-amin und seine Additionssalze.

5. Therapeutische Zusammensetzung, dadurch gekennzeichnet, daß sie in Verbindung mit einem physiologisch annehmbaren Füllstoff mindestens ein Derivat von N-(Methoxyphenacyl)-alkylamin der Formel I nach einem der Ansprüche 1 bis 4, welches aus der aus N-(3-Methoxyphenacyl)-tertiobutylamin, N-(2-Methoxyphenacyl)-tertiobutylamin, N-(4-Methoxyphenacyl)-N-2(2-hydroxy-methyl-propyl)-amin und ihren nichttoxischen Additionssalzen gebildeten Gruppe ausgewählt ist, enthält.

6. Verwendung eines aus der aus
a) N-(3-Methoxyphenacyl)-tertiobutylamin,
b) N-(2-Methoxyphenacyl)-tertiobutylamin,
c) N-(4-Methoxyphenacyl)-N-2-(2-hydroxymethyl-propyl)-amin und
d) ihren nichttoxischen Salzen

gebildeten Gruppe ausgewählten Derivates von N-(Methoxyphenacyl)-alkylamin zur Herstellung eines peripherisch vasodilatatorischen Medikaments zur therapeutischen Anwendung gegen Kreislaufbeschwerden.

7. Verwendung nach Anspruch 6, dadurch gekennzeichnet, daß das Derivat von N-(Methoxyphenacyl)-alkylamin das Methansulfonat von N-(3-Methoxyphenacyl)-tertiobutylamin ist.

8. Verfahren zur Herstellung eines Derivats von N-(Methoxyphenacyl)-alkylamin der Formel I nach Anspruch 1, welches aus der aus

a) N-(3-Methoxyphenacyl)-tertiobutylamin,
b) N-(2-Methoxyphenacyl)-tertiobutylamin,
c) (4-Methoxyphenacyl)-N-2-(2-hydroxymethyl-propyl)-amin und
d) ihren Additionssalzen

gebildeten Gruppe ausgewählt ist, dadurch gekennzeichnet, daß ein Methoxyphenacylbromid der Formel

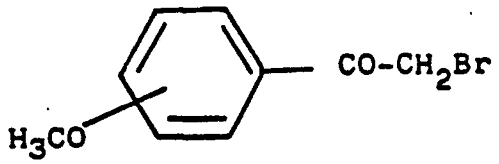 (II)

mit einem Alkylamin der Formel

$H_2NR$ (III)

(worin R die obenangegebene Bedeutung hat) in Gegenwart eines inerten Lösungsmittels, insbesondere ausgewählt aus der aus den $C_1$-$C_2$-Alkoholen, Chloroform, Dioxan und Tetrahydrofuran gebildeten Gruppe, während 0,25 bis 2 h bei einer Temperatur zwischen der Raumtemperatur (15 bis 20°C) und der Rückflußtemperatur des Reaktionsmediums im Verhätlnis von mindestens 3 Molen III zu einem Mol I umgesetzt wird.

**Patentansprüche**

für den Vertragsstaat: AT.

1. Verfahren zur Herstellung eines Derivats von N-(Methoxyphenacyl)-alkylamin der Formel

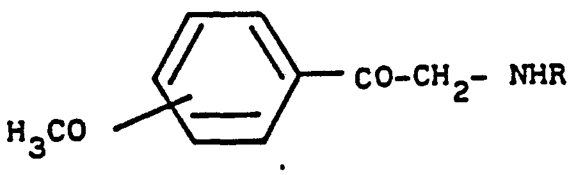 (I),

worin R eine $C(CH_3)_3$- oder $C(CH_3)_2CH_2OH$ bedeutet, welches aus der aus

a) N-(3-Methoxyphenacyl)-tertiobutylamin,
b) N-)2-Methoxyphenacyl)-tertiobutylamin,
c) N-(4-Methoxyphenacyl)-N-2-(2-hydroxymethyl-propyl)-amin und
d) ihren Additionssalzen

gebildeten Gruppe ausgewählt ist, dadurch gekennzeichnet, daß ein Methoxyphenacylbromid der Formel

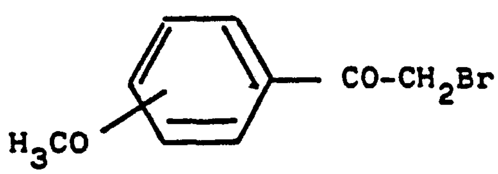 (II)

mit einem Alkylamin der Formel

$H_2NR$ (III)

(worin R die obenangegebene Bedeutung hat) in Gegenwart eines inerten Lösungsmittels, insbesondere ausgewählt aus der aus den $C_1$-$C_2$-Alkoholen, Chloroform, Dioxan und Tetrahydrofuran gebildeten Gruppe, während 0,25 bis 2 h bei einer Temperatur zwischen der Raumtemperatur (15 bis 20°C) und der Rückflußtemperatur des Reaktionsmediums im Verhältnis von mindestens 3 Molen III zu einem Mol I umgesetzt wird.

2. Verfahren nach Anspruch 1 zur Herstellung von N-(3-Methoxyphenacyl)-tertiobutylamin, dadurch gekennzeichnet, daß man 3-Methoxyphenacylbromid mit Tertiobutylamin umsetzt.

**Claims**

for the contracting states: BE, CH, DE, FR, GB, IT, LI, LU, NL, SE.

1. A derivative belonging to the family of N-(methoxyphenacyl)-alkylamines of the formula

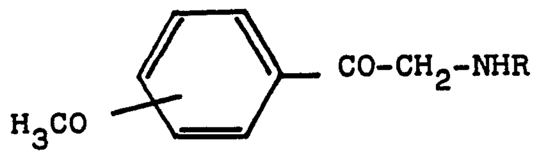

(I)

wherein R represents $C(CH_3)_3$ or $C(CH_3)_2CH_2OH$, characterized in that it is selected from the group consisting of
a) N-(3-methoxyphenacyl)-tert.-butylamine,
b) N-(2-methoxyphenacyl)-tert.-butylamine,
c) N-(4-methoxyphenacyl)-N-2-(2-hydroxymethyl-propyl)-amine, and
d) addition salts thereof.

2. N-(3-methoxyphenacyl)-tert.-butylamine methanesulfonate.

3. N-(2-methoxyphenacyl)-tert.-butylamine and addition salts thereof.

4. N-(4-methoxyphenacyl)-N-2-(2-hydroxymethyl-propyl)-amine and addition salts thereof.

5. A therapeutical composition characterized in that it comprises, in association with a physiologically acceptable excipient, at least an N-(methoxyphenacyl)-alkylamine derivative of the formula I according to anyone of claims 1-4 which is selected from the group consisting of N-(3-methoxyphenacyl)-tert.-butylamine, N-(2-methoxyphenacyl)-tert.-butylamine, N-(4-methoxyphenacyl-N-2-(2-hydroxymethyl-propyl)-amine and non-toxic addition salts thereof.

6. The use of an N-(methoxyphenacyl)-alkylamine derivative selected from the group consisting of
a) N-(3-methoxyphenacyl)-tert.-butylamine,
b) N-(2-methoxyphenacyl)-tert.-butylamine,
c) N-(4-methoxyphenacyl)-N-2-(2-hydroxymethyl-propyl)-amine, and
d) non-toxic addition salts thereof,
for obtaining a peripheral vasodilating medicament destined for a therapeutical indication vis-à-vis circulatory disorders.

7. The use according to claim 6 in which the N-(methoxyphenacyl)-alkylamine derivative is N-(3-methoxyphenacyl)-tert.-butylamine methanesulfonate.

8. A method for preparing a N-(methoxyphenacyl)-alkylamine derivative of the formula I according to claim 1 which is selected from the group consisting of
a) N-(3-methoxyphenacyl)-tert.-butylamine,
b) N-(2-methoxyphenacyl)-tert.-butylamine,
c) N-(4-methoxyphenacyl)-N-2-(2-hydroxymethyl-propyl)-amine, and
d) addition salts thereof,
said method being characterized in that a methoxyphenacyl bromide of the formula

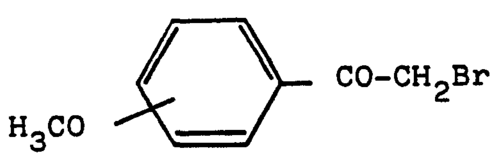

(II)

is reacted with an alkylamine of the formula
$H_2NR$ (III)
(wherein R is defined as indicated above), in the presence of an inert solvent selected from the group comprising $C_1$-$C_2$-alcohols, dioxane and tetrahydrofuran, for a duration of between 0.25 and 2 h, at a temperature comprised between the room temperature (15-20°C) and the reflux temperature of the reaction medium, at a rate of at least 3 mols of III for 1 mol of II.

**Claims**

for the contracting state: AT.

1. A method for preparing an N-(methoxyphenacyl)-alkylamine derivative of the formula

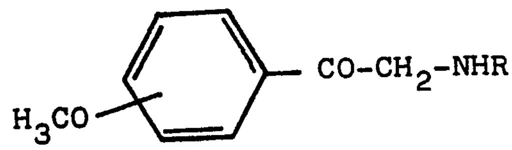

(I)

wherein R represents $C(CH_3)_3$ or $C(CH_3)_2CH_2OH$, said derivative being selected from the group consisting of
a) N-(3-methoxyphenacyl)-tert.-butylamine,
b) N-(2-methoxyphenacyl)-tert.-butylamine,
c) N-(4-methoxyphenacyl)-N-2-(2-hydroxymethyl-propyl)-amine, and
d) addition salts thereof,
said method being characterized in that a methoxyphenacyl bromide of the formula

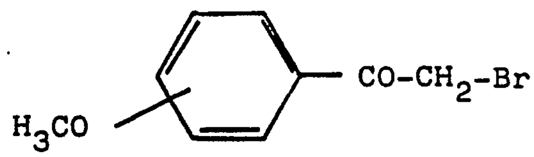

(II)

is reacted with an alkylamine of the formula
$H_2NR$ (III)
(wherein R is defined as indicated above), in the presence of an inert solvent selected from the group comprising $C_1$-$C_2$-alcohols, dioxane and tetrahydrofuran, for a duration of between 0.25 and 2 h, at a temperature comprised between the room temperature (15-20° C) and the reflux temperature of the reaction medium, at a rate of at least 3 mols of III for 1 mol of II.

2. A method according to claim 1 for preparing N-(3-methoxyphenacyl)-tert.-butylamine, characterized in that 3-methoxyphenacyl bromide is reacted with tert.butylamine.